# EUROPEAN PATENT APPLICATION

(11) **EP 1 535 892 A1**
(43) Date of publication of application: **01.06.2005**
(21) Application number: 03741187.3
(22) Date of filing: 03.07.2003
(51) Int. Cl.: C07C 43/17, C07C 41/06

(54) **FLUORINATED UNSATURATED COMPOUND AND PROCESS FOR PRODUCING THE SAME**

(30) Priority: 03.07.2002 JP 2002194887
(71) Applicant: ASAHI GLASS COMPANY LTD., Tokyo 100-8405 (JP)
(72) Inventor: FURUKAWA, Yutaka c/o Asahi Glass Company, Limited, Yokohama-shi, Kanagawa 221-8755 (JP)
(74) Representative: Müller-Boré & Partner Patentanwälte
(86) International application number: PCT/JP2003/008496
(87) International publication number: WO 2004/005231

(57) **Abstract**

A novel fluorine-containing unsaturated compound and a method for its production.

A fluorine-containing unsaturated compound represented by R¹CY¹HCY²Y³OQ¹CH=CH₂ or CH₂=CHQ²OCZ¹Z²CZ³HR²CZ⁴HCZ⁵Z⁶OQ²CH=CH₂ (wherein R¹ is a monovalent fluorine containing organic group, etc., R² is a bivalent fluorine containing organic group, etc., Y¹ to Y³ and Z¹ to Z⁶ are fluorine atoms, and Q¹ and Q² are alkylene groups, etc.). A method for producing a fluorine-containing unsaturated compound having a group represented by -CX¹HCX²X³OQCH=CH₂ (wherein X¹ to X³ are fluorine atoms, and Q is an alkylene group, etc.), which comprises reacting a compound having -CX¹=CX²X³ with HOQCH=CH₂ in the presence of an alkali metal compound.

## Description

### TECHNICAL FIELD

The present invention relates to a novel fluorine-containing unsaturated compound and method for its production.

### BACKGROUND ART

As a fluorine-containing unsaturated compound having -CH=CH₂ at one end, R^{f}(CF₂CF₂)ᵣ(CH₂)ₙCH=CH₂ (R^{f} is a polyfluoroalkyl group, r is an integer of at least 1, and n is an integer of at least 0) may, for example, be mentioned, and the fluorine-containing unsaturated compound is known to be produced by the following methods.

A method which comprises telomerizing R^{f}I and CF₂=CF₂ into R^{f}(CF₂CF₂)ᵣI, adding it to ethylene and converting the resulting R^{f}(CF₂CF₂)ᵣCH₂CH₂I to R^{f}(CF₂CF₂)ᵣCH=CH₂ by using an alkali metal hydroxide such as NaOH.

A method which comprises adding R^{f}(CF₂CF₂)ᵣI to CH₃COOCH₂CH=CH₂ and converting the resulting R^{f}(CF₂CF₂)ᵣCH₂CHICH₂OCOCH₃ to R^{f}(CF₂CF₂)ᵣCH₂CH=CH₂ by using zinc.

However, because R^{f}(CF₂CF₂)ᵣI produced by telomerization is a compound having a distribution in respect of the number of carbon atoms (i.e., r in the above chemical formula), R^{f}(CF₂CF₂)ᵣ(CH₂)ₙCH=CH₂ derived from the compound is also a fluorine-containing compound having a distribution in respect of the number of carbon atoms. Accordingly, separation is required after the reaction in order to obtain a fluorine-containing compound having -CH=CH₂ at one end and a specific number of carbon atoms.

The present inventors found that it is necessary to use HOQCH=CH₂ (wherein Q represents a bivalent organic group) as a starting material for the reaction in order to solve the problem of the distribution in respect of the number of carbon atoms in the above telomerization reaction, and found a novel compound having -CH=CH₂ at one end based on the knowledge.

Namely, the object of the present invention is to provide a novel fluorine-containing unsaturated compound and a method for its production.

### DISCLOSURE OF THE INVENTION

The present invention provides a fluorine-containing unsaturated compound represented by the following formula 1 (compound 1) or the following formula 2 (compound 2):

R¹CY¹HCY²Y³OQ¹CH=CH₂ Formula 1

CH₂=CHQ²OCZ¹Z²CZ³HR²CZ⁴HCZ⁵Z⁶OQ³CH=CH₂ Formula 2

wherein the symbols in the formulae (1) and (2) have the following meanings:
R¹: a monovalent organic group, a halogen atom or a hydrogen atom;
R²: a bivalent organic group;
Y¹, Y² and Y³: independently hydrogen atoms or fluorine atoms provided that when R¹ is not a fluorine atom, at least one of Y¹, Y² and Y³ is a fluorine atom;
Z¹, Z², Z³, Z⁴, Z⁵ and Z⁶: independently hydrogen atoms or fluorine atoms provided that at least one of Z¹, Z² and Z³ is a fluorine atom, and at least one of Z⁴, Z⁵ and Z⁶ is a fluorine atom;
Q¹, Q² and Q³: independently bivalent organic groups.

Further, the present invention provides a method for producing a fluorine-containing unsaturated compound (compound 5) having a group represented by the following formula (5), which comprises reacting a compound (compound 3) having a group represented by the following formula (3) and a compound (compound 4) represented by the following formula (4) in the presence of an alkali metal compound:

-CX¹=CX²X³ Formula 3

HOQCH=CH₂ Formula 4

-CX¹HCX²X³OQCH=CH₂ Formula 5

wherein the symbols in the formulae (3), (4) and (5) have the following meanings:
X¹, X² and X³: independently hydrogen atoms or fluorine atoms provided that at least one of X¹, X² and X³ is a fluorine atom;
Q: a bivalent organic group.

### BEST MODE FOR CARRYING OUT THE INVENTION

In compound 1, R¹ is a monovalent organic group, a halogen atom or a hydrogen atom. R¹ is preferably a monovalent hydrocarbon group or a monovalent halogenated hydrocarbon group, more preferably a monovalent halogenated hydrocarbon group containing an etheric oxygen atom. R¹ may be linear, branched or cyclic.

R¹ is preferably a fluorohydrocarbon group, more preferably be a perfluorohydrocarbon group having all the hydrogen atoms of a hydrocarbon group substituted by fluorine atoms, and most preferably a perfluorohydrocarbon group containing an etheric oxygen atom which, if any, is preferably present at a terminal portion.

In the compound 1, Y¹, Y² and Y³ are preferably fluorine atoms, and it is preferred that all of them are fluorine atoms. Further, Q¹ is preferably a bivalent organic group, more preferably an alkylene group. Further, Q¹ is preferably a group represented by -(CH₂)ₜ- (t is an integer of at least 1), and t is preferably from 1 to 12, particularly preferably from 1 to 6.

The compound 1 is preferably a compound (compound 6) represented by R'CFHCF₂O(CH₂)ₘCH=CH₂ (wherein R' is a monovalent fluorine-containing C₁₋₁₆ organic group containing an etheric oxygen atom, and m is an integer of from 1 to 12) in view of ease of synthesis. In the compound 6, R' may be linear or branched, and if branched, preferably has a branch preferably represented by (CF₃)₂CF- at one end.

R' is preferably a fluorinated hydrocarbon group, more preferably a perfluorohydrocarbon group having all the hydrogen atoms of a hydrocarbon group substituted by fluorine atoms, most preferably a perfluorohydrocarbon group containing an etheric oxygen atom which, if any, is preferably present at one end.

Specific examples of the compound 1 are the following compounds.
HCF₂CF₂OCH₂CH=CH₂, CF₃CFHCF₂OCH₂CH=CH₂, CF₃CFHCF₂OCH₂CH₂CH=CH₂, CF₃CFHCF₂OCH₂(CH₂)₂CH=CH₂, CF₃CFHCF₂OCH₂(CH₂)₃CH=CH₂, C₂F₅CFHCF₂OCH₂CH=CH₂, C₂F₅CFHCF₂OCH₂CH₂CH=CH₂, C₂F₅CFHCF₂OCH₂(CH₂)₂CH=CH₂, C₂F₅CFHCF₂OCH₂(CH₂)₃CH=CH₂, C₃F₇CFHCF₂OCH₂CH=CH₂, C₃F₇CFHCF₂OCH₂CH₂CH=CH₂, C₃F₇CFHCF₂OCH₂(CH₂)₂CH=CH₂, C₃F₇CFHCF₂OCH₂(CH₂)₃CH=CH₂, C₅F₁₁CFHCF₂OCH₂CH=CH₂, C₅F₁₁CFHCF₂OCH₂CH₂CH=CH₂, C₅F₁₁CFHCF₂OCH₂(CH₂)₂CH=CH₂, C₅F₁₁CFHCF₂OCH₂(CH₂)₃CH=CH₂, C₇F₁₅CFHCF₂OCH₂CH=CH₂, C₇F₁₅CFHCF₂OCH₂CH₂CH=CH₂, C₇F₁₅CFHCF₂OCH₂(CH₂)₂CH=CH2, C₇F₁₅CFHCF₂OCH₂(CH₂)₃CH=CH₂.
(CF₃)₂CFCF₂CFHCF₂OCH₂CH=CH₂, (CF₃)₂CFCF₂CFHCF₂OCH₂CH₂CH=CH₂, (CF₃)₂CFCF₂CFHCF₂OCH₂(CH₂)₂CH=CH₂, (CF₃)₂CFCF₂CFHCF₂OCH₂(CH₂)₃CH=CH₂.
C₃F₇OCFHCF₂OCH₂CH=CH₂, C₃F₇OCFHCF₂OCH₂CH₂CH=CH₂, C₃F₇OCFHCF₂OCH₂(CH₂)₂CH=CH₂, C₃F₇OCFHCF₂OCH₂(CH₂)₃CH=CH₂, C₄F₉OCFHCF₂OCH₂CH=CH₂, C₄F₉OCFHCF₂OCH₂CH₂CH=CH₂, C₄F₉OCFHCF₂OCH₂(CH₂)₂CH=CH₂, C₄F₉OCFHCF₂OCH₂(CH₂)₃CH=CH₂, C₅F₁₁OCFHCF₂OCH₂CH=CH₂, C₅F₁₁OCFHCF₂OCH₂CH₂CH=CH₂, C₅F₁₁OCFHCF₂OCH₂(CH₂)₂CH=CH₂, C₅F₁₁OCFHCF₂OCH₂(CH₂)₃CH=CH₂, C₆F₁₃OCFHCF₂OCH₂CH=CH₂, C₆F₁₃OCFHCF₂OCH₂CH₂CH=CH₂ C₆F₁₃OCFHCF₂OCH₂(CH₂)₂CH=CH₂, C₆F₁₃OCFHCF₂OCH₂(CH₂)₃CH=CH₂.
[CF(CF₃)CF₂O]CFHCF₂OCH₂CH=CH₂, F[CF(CF₃)CF₂O]CFHCF₂OCH₂(CH₂)₃CH=CH₂, F[CF(CF₃)CF₂O]₂CFHCF₂OCH₂CH=CH₂, F[CF(CF₃)CF₂O]₂CFHCF₂OCH₂(CH₂)₃CH=CH₂, F[CF(CF₃)CF₂O]₃CFHCF₂OCH₂CH=CH₂ and F[CF(CF₃)CF₂O]₃CFHCF₂OCH₂(CH₂)₃CH=CH₂.

In the compound 2, R² is preferably a bivalent hydrocarbon group or a bivalent halogenated hydrocarbon group, more preferably a bivalent halogenated hydrocarbon group containing an etheric oxygen atom. R² may be linear, branched or cyclic.

R² is preferably a fluorinated hydrocarbon group, more preferably a perfluorohydrocarbon group having all the hydrogen atoms of a hydrocarbon group substituted by fluorine atoms, most preferably a perfluorohydrocarbon group containing an etheric oxygen atom which, if any, is preferably present at a terminal portion.

In the compound 2, Z¹, Z², Z³, Z⁴, Z⁵ and Z⁶ are preferably fluorine atoms, and it is preferred that all of them are fluorine atoms. Further, Q² and Q³ are preferably the same as mentioned for Q¹ in the above compound 1.

The compound 2 is preferably a compound (compound 7) represented by R" [CFHCF₂O(CH₂)_{q}CH=CH₂]₂ (wherein R'' is a bivalent fluorine-containing C₁₋₁₆ organic group containing an etheric oxygen atom, and q is an integer of from 1 to 12) in view of ease of synthesis. In the compound 7, R'' may be linear or branched, and if branched, preferably has a branch preferably represented by (CF₃)₂CF- at one end.

R'' is preferably a fluorinated hydrocarbon group, more preferably a perfluorohydrocarbon group having all the hydrogen atoms of a hydrocarbon group substituted by fluorine atoms, most preferably a perfluorohydrocarbon group containing an etheric oxygen atom which, if any, is preferably present at a terminal portion.

Specific examples of the compound 2 are the following compounds.
CH₂=CHCH₂OCF₂CFH(CF₂)₂CFHCF₂OCH₂CH=CH₂, CH₂=CHCH₂CH₂OCF₂CFH(CF₂)₂CFHCF₂OCH₂CH₂CH=CH₂, CH₂=CH (CH₂)₃CH₂OCF₂CFH(CF₂)₂CFHCF₂OCH₂(CH₂)₃CH=CH₂, CH₂=CHCH₂OCF₂CFH[OCF₂CF(CF₃)]OCF₂(CF₂)₄CF₂O[CF(CF₃)CF₂O]CFHC F₂OCH₂CH=CH₂ and
CH₂=CH (CH₂)₃CH₂OCF₂CFH[OCF₂CF(CF₃)]OCF₂(CF₂)₄CF₂O[CF(CF₃)CF₂O ]CFHCF₂OCH₂(CH₂)₃CH=CH₂.

In the compound 3, X¹, X² and X³ are preferably fluorine atoms, and it is preferred that all of them are fluorine atoms. The compound 3 can be prepared by the method disclosed in J. Am. Chem. Soc, 75, 4525 (1953) or the like.

The compound 3 is preferably a compound (compound 6) represented by the following formula 6.

R[CX¹=CX²X³]ₚ Formula 6

R: a p-valent organic group;
p: an integer of from 1 to 4;
X¹, X² and X³: independently hydrogen atoms or fluorine atoms provided that at least one of X¹, X² and X³ is a fluorine atom.

In the compound 6, R is preferably the same as mentioned for R¹ in the compound 1 or for R² in the compound 2. X¹, X² and X³ are preferably fluorine atoms, and it is preferred that all of them are fluorine atoms. p is preferably 1 or 2.

Specific examples of the compound 3 are the following compounds.
CF₂=CF₂, CF₃CF=CF₂, C₂F₅CF=CF₂, C₃F₇CF=CF₂, C₅F₁₁CF=CF₂, C₇F₁₅CF=CF₂, C₃F₇OCF=CF₂, C₄F₉OCF=CF₂, C₅F₁₁OCF=CF₂, C₆F₁₃OCF=CF₂, (CF₃)₂CFCF₂CF=CF₂, F[CF(CF₃)CF₂O]CF=CF₂, F[CF(CF₃)CF₂O]₂CF=CF₂, F[CF(CF₃)CF₂O]₃CF=CF₂.
CF₂=CF(CF₂)₂CF=CF₂, CF₂=CF(CF₂)₃CF=CF₂, CF₂=CFO(CF₂)₂OCF=CF₂, CF₂=CFO(CF₂)₃OCF=CF₂ and CF₂=CF[OCF₂CF(CF₃)]OCF₂(CF₂)₄CF₂O[CF(CF₃)CF₂O]CF=CF2.

In the compound 4, Q is preferably the same as mentioned for Q¹ in the compound 1.

As specific examples of the compound 4, HOCH₂CH=CH₂, HO(CH₂)₂CH=CH₂, HO(CH₂)₃CH=CH₂, HO(CH₂)₄CH=CH₂ and HO(CH₂)₆CH=CH₂ are preferably mentioned.

In the compound 5, X¹, X², X³ and Q are preferably the same as those in the compound 3 or 4.

The compound 5 is preferably a compound (compound 7) represented by the following formula 7.

R[CX¹HCX²X³OQCH=CH2]ₚ Formula 7

R: a p-valent organic group;
X¹, X² and X³: independently hydrogen atoms or fluorine atoms provided that at least one of X¹, X² and X³ is a fluorine atom;
Q: a bivalent organic group;
P: an integer of from 1 to 4.

In the compound 7, R, X¹, X², X³, Q and p are preferably the same as those in the compound 4 or the compound 6. The compound 5 is preferably the compound 1 or the compound 2.

The alkali metal compound to be used in the reaction of the compound 3 and the compound 4 is preferably an alkali metal, an alkali metal hydride, an alkali metal hydroxide, an alkali metal amide or the like. Specifically, an alkali metal such as Na, K or Cs, an alkali metal hydroxide such as NaOH or KOH, an alkali metal hydride such as NaH or KH, or an alkali metal amide such as NaNH₂ or KNH₂ is preferably mentioned.

In the above reaction, the alkali metal is preferably used in an amount of from 0.01 to 1 mol, particularly preferably in an amount of from 0.05 to 0.5 mol in view of the reaction rate, based on 1 mol of the compound 3, though there is no particular restriction. The usage range is preferred because the reaction proceeds at an appropriate reaction rate with little production of by-products.

In the reaction, the compound 4 is preferably used in an amount of from 1 to 1.5 mols, more preferably from 1.01 to 1.1 mols based on 1 mol of the compound 3 for production of the compound having a group represented by the formula 5, namely the compound 1, and is preferably used in an amount of from 2 to 3 mols, more preferably from 2.02 to 2.2 mols based on 1 mol of the compound 3 for production of the compound having two groups represented by the formula 5, namely the compound 2.

Further, the temperature for the reaction is preferably from 0 to 150°C, more preferably from 40 to 120°C. The reaction is preferably carried out within this temperature range because the reaction proceeds at an appropriate reaction rate without homopolymerization of the compound 3.

Further, in the reaction, a solvent may or may not be used, but it is preferably used. The solvent is preferably a solvent which dissolves the compound 4, a solvent which dissolves the compound 5, or a solvent which is practically inert in the reaction. The solvent is preferably an ether, a nitrile compound or the like, and specifically, it is preferably diethyl ether, glyme, dioxane, tetrahydrofuran, acetonitrile or propionitrile, more preferably dioxane, tetrahydrofuran or acetonitrile.

The solvent is preferably used in such an amount that the compound 5 as the product accounts for from 1 to 60 mass%, more preferably from 3 to 50 mass% in view of the reaction rate and the productivity, though there is no particular restriction.

Further, the reaction can be carried out in a two layer system consisting of an organic solvent and an aqueous alkali metal hydroxide solution in the presence of a phase-transfer catalyst. The organic solvent may be an aliphatic hydrocarbon such as hexane or heptane; an alicyclic hydrocarbon such as cyclohexane; an aromatic hydrocarbon such as benzene, toluene or xylene; a halogenated hydrocarbon such as chloroform, dichloromethane, tetrachloromethane or perfluorohexane; an ether such as tetrahydrofuran; a ketone such as acetone or methylethyl ketone; or an organic solvent such as acetonitrile, DMF or DMSO. The phase-transfer catalyst may, for example, be tetrabutylammonium chloride or tetrabutylammonium bromide. As an aqueous alkaline solution, the phase-transfer catalyst is preferably used in an amount of approximately from 0.01 to 1 mol based on 1 mol of the compound 5. The aqueous alkali metal hydroxide solution preferably has a concentration of from 10 to 50 mass%. The reaction temperature is selected within the range between room temperature and the reflux temperature of the solvent.

Further, the compound 3 tends to polymerize at high pressure, and therefore in order to prevent the polymerization, the polymerization inhibitor is preferably used in the reaction. The polymerization inhibitor may be put in a reaction system before or with the starting materials. The polymerization inhibitor is preferably limonene, pinene, cymene, terpinene or the like, though there is no particular restriction.

In the present invention, the compound 5 is presumed to be produced by the following mechanism.

Namely, the alkali metal compound converts HOCH₂QCH=CH₂ (compound 4) to an alkoxide compound, the alkoxide compound is added to the compound 3, and then the metal portion is replaced by a hydrogen atom to produce the compound 5. The use of the compound 4 having a hydroxyl group and an ethylenically unsaturated group allows production of a fluorine-containing unsaturated compound because an ethylenically unsaturated group having fluorine atoms in the compound 3 reacts with the hydroxyl group, while the other ethylenically unsaturated group remains.

According to the present invention, a novel fluorine-containing unsaturated compound can be produced. The fluorine-containing unsaturated compound is useful as a monomer for preparing various polymers and can be polymerized by using a metallocene catalyst or the like. Further, it is useful as an intermediate of various compounds and can be reacted with various silicon compound.

For example, R¹CY¹HCY²Y³OQ¹CH₂CH₂Si(CH₃)₃₋ₙXₙ obtained by reacting the compound 1 with H-Si(CH₃)₃₋ₖXₖ (k is from 1 to 3, and X is a hydrolyzable group such as a halogen atom or an alkoxy group) can be used as a silane coupling agent, is useful as a surface treatment for glass, metal or a powder and can be reacted with a siloxane polymer having a H-Si(CH₃)O_{2/2} group to obtain a fluorine-containing silicone oil.

Further, the compound 3 as the starting material for the fluorine-containing unsaturated compound of the present invention can be prepared by the direct fluorination disclosed in Adv. Synth. Catal. 2001, 343, No. 2, which allows a choice of the structure of the starting material and therefore can give fluorine-containing unsaturated compounds in various structures.

### EXAMPLES

Now, the present invention will be described in detail with reference to Examples, but it should be understood that the present invention is by no means restricted thereto.

### EXAMPLE 1

30 g of 1,4-dioxane, 10 g of C₃F₇OCF=CF₂, 4.0 g of HO(CH₂)₄CH=CH₂ and 0.55 g of KOH were sealed in a stainless steel reaction vessel having a capacity of 50 mL, reacted at 70°C for 8 hours with stirring, and after addition of 50 mL of water, allowed to separate into two layers. The organic layer was distilled to obtain 11.1 g of C₃F₇OCFHCF₂O(CH₂)₄CH=CH₂ as the product.

The results of identification of the product are shown below.
IR (neat): 1643, 1341, 1238, 1199, 1154, 1095 cm⁻¹,
¹H-NMR(CDCl₃)δ: 1.43 - 1.57 (m, 2H, OCH₂CH₂), 1.64 - 1.78 (m, 2H, CH₂CH₂CH=CH₂), 2.05 - 2.15 (m, 2H, CH₂CH=CH₂), 3.99 (t, J=6.4 Hz, 2H, OCH₂), 4.95 - 5.09 (m, 2H, =CH₂), 5.72 - 5.88 (m, 1H, CH=), 5.85 (d, t, 53.7 Hz, 2.9 Hz, 1H, CFHCF₂),
¹⁹F-NMR(CDCl₃)δ: -81.4 (t, J=7.5 Hz, 3F, CF₃), -84.9 and -87.0 (ABquartet, J=145 Hz, 2F, CF₂OCFH), -89.2 and -90.0 (ABquartet, J=142 Hz, 2F, CF₂OCH₂), -129.5 - -129.6 (m, 2F, CF₂CF₃), -144.2 (d, quintet, J=53.7 Hz, 7.5 Hz, 1F, CFH).

### EXAMPLE 2

150 g of hexane, 50 g of C₃F₇OCF=CF₂, 19.8 g of HO(CH₂)₄CH=CH₂, 150 g of a 50% aqueous sodium hydroxide and 600 mg of (n-C₄H₉)₄NBr were sealed in a stainless steel reaction vessel having a capacity of 500 mL, reacted at room temperature for 8 hours with stirring, and after addition of 500 mL of water, allowed to separate into two layers. The organic layer was distilled to obtain 61.9 g of C₃F₇OCFHCF₂O(CH₂)₄CH=CH₂ as the product.

### EXAMPLE 3

30 g of 1,4-dioxane, 10 g of CF₂=CFO(CF₂)₂OCF=CF₂, 7.15 g of HO(CH₂)₄CH=CH₂ and 0.55 g of KOH were sealed in a stainless steel reaction vessel having a capacity of 50 mL, reacted at 70°C for 8 hours with stirring, and after addition of 50 mL of water, allowed to separate into two layers. The organic layer was distilled to obtain 11.39 g of CH₂=CH(CH₂)₄OCF₂HCFO(CF₂)₂OCFHCF₂O(CH₂)₄CH=CH₂.

The results of identification of the product are shown below.
IR(neat): 1643, 1341, 1238, 1199, 1154, 1095 cm⁻¹,
¹H-NMR(CDCl₃)δ: 1.43 - 1.57 (m, 4H, OCH₂CH₂), 1.64 - 1.78 (m, 4H, CH₂CH₂CH=CH₂), 2.05 - 2.15 (m, 4H, CH₂CH=CH₂), 3.99 (t, J=6.4 Hz, 4H, OCH₂), 4.95 - 5.09 (m, 4H, =CH₂), 5.72 - 5.88 (m, 2H, CH=), 5.85 (d, t, 53.7 Hz, 2.9 Hz, 2H, CFHCF₂),
¹⁹F-NMR (CDCl₃)δ: -84.86 and -86.96 (ABquartet, J=145.0 Hz, 4F, CF₂OCFH), -89.30 and -89.94 (ABquartet, J=144.0 Hz, 4F, CF₂OCH₂), -144.2 (d, quintet, J=53.7 Hz, 8.6 Hz, 2F, CFH).

## Claims

1. A fluorine-containing unsaturated compound represented by the following formula (1) or (2):
R¹CY¹HCY²Y³OQ¹CH=CH₂ Formula 1
CH₂=CHQ²OCZ¹Z²CZ³HR²CZ⁴HCZ⁵Z⁶OQ³CH=CH₂ Formula 2
wherein the symbols in the formulae (1) and (2) have the following meanings:
R¹: a monovalent organic group, a halogen atom or a hydrogen atom;
R²: a bivalent organic group;
Y¹, Y² and Y³: independently hydrogen atoms or fluorine atoms provided that when R¹ is not a fluorine atom, at least one of Y¹, Y² and Y³ is a fluorine atom;
Z¹, Z², Z³, Z⁴, Z⁵ and Z⁶: independently hydrogen atoms or fluorine atoms provided that at least one of Z^{1,} Z² and Z³ is a fluorine atom, and at least one of Z⁴, Z⁵ and Z⁶ is a fluorine atom;
Q¹, Q² and Q³: independently bivalent organic groups.

2. A method for producing a fluorine-containing unsaturated compound having a group represented by the following formula (5), which comprises reacting a compound having a group represented by the following formula (3) and a compound represented by the following formula (4) in the presence of an alkali metal compound:
-CX¹=CX²X³ Formula 3
HOQCH=CH₂ Formula 4
-CX¹HCX²X³OQCH=CH₂ Formula 5
wherein the symbols in the formulae (3), (4) and (5) have the following meanings:
X¹, X² and X³: independently hydrogen atoms or fluorine atoms provided that and at least one of X¹, X² and X³ is a fluorine atom;
Q: a bivalent organic group.

3. The fluorine-containing unsaturated compound according to Claim 1, wherein R¹ in the formula (1) is a monovalent perfluorohydrocarbon group containing an etheric oxygen atom, and R² in the formula (2) is a bivalent perfluorohydrocarbon group containing an etheric oxygen atom.

4. The fluorine-containing unsaturated compound according to Claim 1, wherein Q¹ in the formula (1), and Q² and Q³ in the formula (2) are -(CH₂)ₜ- (t is an integer of at least 1).

5. The fluorine-containing unsaturated compound according to Claim 1, wherein the compound represented by the formula (1) is R'CFHCF₂O(CH₂)ₘCH=CH₂ (wherein R' is a monovalent fluorine-containing C₁₋₁₆ organic group containing an etheric oxygen atom, and m is an integer of from 1 to 12), and the compound represented by the formula (2) is R" [CFHCF₂O(CH₂)_{q}CH=CH₂]₂ (wherein R" is a bivalent fluorine-containing C₁₋₁₆ organic group containing an etheric oxygen atom, and q is an integer of from 1 to 12).

6. The fluorine-containing unsaturated compound according to Claim 2, wherein Q in the formula (4) or (5) is -(CH₂)ₜ- (t is an integer of at least 1).

7. The method for producing a fluorine-containing unsaturated compound according to Claim 2, wherein the compound represented by the formula (3) is a compound represented by the following formula (6).
R[CX¹=CX²X³]ₚ Formula 6
wherein the symbols in the formula (6) have the following meanings:
R: a p-valent organic group;
p: an integer of from 1 to 4;
X¹, X² and X³: independently hydrogen atoms or fluorine atoms provided that at least one of X¹, X² and X³ is a fluorine atom:

8. The method for producing a fluorine-containing unsaturated compound according to Claim 2 or 7, wherein the reaction is carried out in a two layer system consisting of an organic solvent and an aqueous alkaline solution in the presence of a phase-transfer catalyst.

9. The method for producing a fluorine-containing unsaturated compound according to Claim 2, 7 or 8, wherein the reaction is carried out in the presence of a polymerization inhibitor.
